Europäisches Patentamt

European Patent Office

Office européen des brevets

(11) Publication number: **0 078 205**
**B1**

# EUROPEAN PATENT SPECIFICATION

(45) Date of publication of patent specification: **04.12.85**

(51) Int. Cl.[4]: **C 12 N 1/10**

(21) Application number: **82401933.5**

(22) Date of filing: **21.10.82**

(54) In vitro production of intracellular protozoa.

(30) Priority: **23.10.81 US 314153**

(43) Date of publication of application:
**04.05.83 Bulletin 83/18**

(45) Publication of the grant of the patent:
**04.12.85 Bulletin 85/49**

(84) Designated Contracting States:
**CH DE FR GB LI NL**

(56) References cited:
**BRITISH JOURNAL OF EXPERIMENTAL
PATHOLOGY, vol. 42, 1961, LONDON (GB) E.
LUND et al.: "Studies of toxoplasma gondii in
cell cultures by means of irradiation
experiments", pages 404-407
JOURNAL OF PARASITOLOGY, vol. 44, no. 2,
1958, LAWRENCE, KANS (US) M. COOK et al.:
"Cultivation of toxoplasma gondii in tissue
cultures of various derivations", pages 172-182
NATURE NEW BIOLOGY, vol. 243, June 20,
1973, LONDON (GB) K.K. SETHI et al.:
"Multiplication of Toxoplasma gondii in
enucleated L cells", pages 255-256**

(73) Proprietor: **MERCK & CO. INC.,
126, East Lincoln Avenue P.O. Box 2000
Rahway New Jersey 07065 (US)**

(72) Inventor: **Schmatz, Dennis M.
3221 Village Drive
Avenel New Jersey 07001 (US)**
Inventor: **Murray, Peter K.
104 Conover Lane
Red Bank New Jersey 07701 (US)**

(74) Representative: **Martin, Jean-Jacques et al
Cabinet REGIMBEAU 26, Avenue Kléber
F-75116 Paris (FR)**

(56) References cited:
**PROCEEDINGS OF THE SOCIETY OF
EXPERIMENTAL BIOLOGY AND MEDICINE, vol.
142, 1973, NEW YORK (US) T.C. JONES:
"Multiplication of Toxoplasmas in enucleate
fibroblasts", pages 1268-1271
EXPERIMENTAL PARASITOLOGY, vol. 52, no.
1, 1981, NEW YORK (US) E.R. PFEFFERKORN et
al.: "Toxoplasma gondii: Growth in the
absence of host cell protein synthesis", pages
129-136**

Courier Press, Leamington Spa, England.

EP 0 078 205 B1

**CHEMICAL ABSTRACTS, vol. 62, no. 4, February 15, 1965, column 4369 d-g, COLUMBUS, Ohio (US) K. SHIMIZU: "Toxoplasmosis. III. Tissue culture of Toxoplasma gondii"**

## Description

Background of the invention

Many species of protozoan parasites which have either a complete or partial intracellular life cycle can be grown *in vitro* in a variety of host cells. Leishmania, Toxoplasma, Plasmodia, Theileria, Anaplasma, Trypanosoma, Coccidia, and Babesia represent protozoal genera where intracellular parasite stages have been successfully grown *in vitro* in host cells.

Some of these parasites are conventionally cultivated in cells which divide rapidly and in consequence one major problem associated with *in vitro* cell culture of protozoa is that host cells may overgrow and die before parasite infections mature. *Plasmodium fallax* grown in embryonic turkey brain cells represents an example of this type. A second problem may occur when host cell division results in the unequal distribution of intracellular parasites in daughter cells. In situations like this, by virtue of an infected host cell dividing into one infected and one uninfected daughter cell, there is a progressive reduction in the proportion of parasite infected cells as the culture interval increases. *Trypanosoma cruzi* grown in normal rat myoblast cells represents an example of this type. A third problem commonly encountered, e.g., in the culture of *Eimeria tenella* in chick kidney explant cells, is after a heterogeneous population of cells is exposed to parasites and only one cell type is infected, the other non-susceptible cell types may divide and overgrow the infected population.

These cell culture problems serve to significantly limit the proportion of parasite infected cells in culture and thus the overall parasite productivity per cultured cell. Secondly, the effect of host cell division on the growth of intracellular protozoa is an important one since, at division parasites may be redistributed unequally between daughter cells. In consequence, after some days a complete spectrum of infection is observed ranging from uninfected cells to cells packed with fully mature parasites. Over the culture period there is therefore a progressive reduction in the percentage of parasitized cells and since the infection is developing asynchronously there is an overall prolongation of the time interval during which parasites are released.

Objects of the invention

It is an object of the present invention to provide stable non-dividing host cells which are susceptible to infection with, and capable of supporting the intracellular development of, protozoan parasites *in vitro*. A further object is to increase the overall percentage of host cells infected by parasites and thus the total parasite yield/cultured host cell. Another object is to synchronize the development of intracellular stages in individual host cells. Still another object is to produce greater amounts of parasite materials than with methods heretofore available.

These and other objects of the present invention will be apparent from the following description.

Summary of the invention

Monolayers of host cells grown in culture (either stationary or in roller flasks) are treated to inhibit cell division. After a subsequent time interval equivalent to the cell division time, these treated host cells are infected with the desired species of protozoa; the number of parasites and duration of exposure of host cells to infection depending on the species and strain of parasite. Culture media is changed every two to three days and extracellular parasites are harvested from the tissue culture supernatant fluid at the end of the intracellular development.

Detailed description of the invention

The present invention relates to the production of intracellular protozoa, except Toxoplasma, and, in particular, to cell cultures which have been rendered incapable of division but which remain susceptible to infection with, and capable of supporting the intracellular growth of intracellular protozoa, except Toxoplasma.

The process of the present invention is applicable to the *in vitro* culture of intracellular protozoa, except Toxoplasma, such as, for example, *Trypanosoma cruzi*, *Eimeria tenella*, and *Plasmodium fallax*. The organisms proceed through their intracellular development normally and yield infective parasites.

The protozoa are grown in a susceptible host cell which is grown in known manner in tissue culture. The cell culture medium and environmental conditions employed are selected based upon the specific host/cell/parasite combination.

Host cells treated to inhibit cell division are left undisturbed post-treatment for a period exceeding the time required for one normal cell division thereby synchronizing all cells at the point of the mitotic block. The cells are then infected with the particular protozoa which it is desired to grow. In general, best results are obtained when the number of infecting parasites exceeds the number of treated host cells. After an exposure period effective for the particular strain of parasite to infect the specific susceptible host cell, all remaining extracellular parasites are aspirated off and the media replaced. (The time of exposure to obtain optimal infection depends on the particular strain and the specific susceptible host cell). By infecting treated host cells it is possible to significantly increase the overall proportion of parasite-infected, compared to non-infected cells in culture; to obtain higher parasite yields under similar temporal conditions than with conventional culture systems; and to improve the overall synchrony of infection.

Parasites produced from cultures of cells treated to inhibit division are still highly infectious. All parasites from these culture systems are morphologically identical to those that were found under natural conditions as well.

The treatment to inhibit cell division may comprise various means such as, for example, physical treatment, such as irradiation, (e.g., gamma radiation, X-rays and ultra violet light), or heat, or by chemical treatment for example, with such agents as colchicine or demecolcine. Irradiation is preferred. The level of radiation employed is that level required to inhibit cell division with minimal cell damage.

The following examples illustrate the present invention without, however, limiting the same thereto.

Example 1

Rat L-6 myoblast cells, $4 \times 10^6$ cells, are seeded in a roller bottle, 850 cm$^2$, using a suitable cell culture medium, for example, 65% Dulbecco's MEM, 20% medium 199, 10% heat inactivated horse serum, and 5% chick embryo extract. After 2 days the cell count has increased to $3.2 \times 10^7$. The roller flask then is exposed to 3,000 rads of gamma radiation from a Cobalt 76 source to inhibit mitosis. After 24 hours the cells are removed by trypsinization, and seeded into four 150 cm$^2$ flasks at a density of $8 \times 10^6$ cells/flask. The cell culture medium consists of 1 part of the above mentioned cell culture medium and 4 parts of Dulbecco's MEM. After the cells have adhered, typically 4—6 hours, they are infected by addition of viable $4 \times 10^7$ *T. cruzi* trypomastigotes, (Merck strain MERC2CD14, passaged 40 times in cell culture in rat L-6 myoblast cells although it is to be understood that any other strain of *T. cruzi* may be used) at a ratio of 5 parasites per irradiated myoblast cell. After infection the medium is removed and replaced on days 1 and 4. Parasites are harvested daily from the culture medium from day 5 to day 7. The total number of parasites collected over the 3 day period is from 8 to $9.5 \times 10^8$ per flask. The parasites are at least as virulent to mice after 40 passages as the starting strain.

Example 2

Rat L-6 myoblast cells, $6 \times 10^6$ are seeded in a roller bottle, 850 cm$^2$ using the same cell culture medium as used in Example 1. After 2 days the cell count has increased to $4.8 \times 10^7$. The roller flask then is exposed to 3000 rads of gamma radiation as in Example 1. Immediately after radiation the media is replaced by the same medium as in Example 1 after irradiation. After 24 hours the cells are infected by addition of $2.4 \times 10^8$ viable *T. cruzi* trypomastigotes. The medium is removed and replaced on days 1 and 4. Parasites are harvested daily from culture medium from day 5 to day 7. The total number of parasites collected over this 3 day period from the roller bottle is from $4.8 \times 10^9$ to $5.8 \times 10^9$. The parasites are at least as virulent to mice after 40 passages as the starting strain.

Example 3

Embryonic turkey brain cells were grown in a media composed of equal volumes of Medium 199 and Basal Media (Eagle) supplemented with 10% fetal bovine sera, 1% penicillin-streptomycin (5,000 units—5000 mcg), 1% glutamine (200 mM/ml), 1% non-essential amino acid solution and 12 mg of Folinic acid. Cells were seeded at a level of $1 \times 10^6$ cells/25 cm$^2$ flask, irradiated with 3000 rads of gamma radiation and infected 24 hours with $1 \times 10^7$ cultured merozoites of *Plasmodium fallax*, all cultures were incubated in 10% CO$_2$ at 37°C. At 96 hours mature segmenting schizonts could be observed and during day 5 merozoites began to emerge. These merozoites were fully capable of reinvading and developing in new host cells. A comparative study showed a 4.5 fold increase in the percentage of infected cells at 96 hours when compared to infections of non-irradiated cells.

Example 4

Primary chick kidney cells and MDBK cells were grown in media composed of Eagles MEM with 10% Fetal Bovine sera, 1% non-essential amino acids, 1% sodium pyrugate, 1% pen-strep-fungizone, and 1% glutamine. Cells were seeded at a level of $1 \times 10^6$ cells/25 cm$^2$ flask, irradiated with 3000 rads of gamma radiation and infected 24 hours later with $5 \times 10^6$ sporozoites of *Eimeria tenella*. Twenty-four hours after infected the media was replaced with Hank's balanced salt solution with 5% fetal bovine serum, 1% glutamine, 1% penicillin-streptomycin (5000 units each) and 2% lactalbumin hydrolysate (10% solution), all cultures were incubated at 5% CO$_2$ and 41°C. Segmenting schizonts could be observed at day 3 post-infection in both cell types and in both cases yielded extracellular merozoites which were capable of initiating secondary infections.

Example 5

A cloned population of MDBK cells (Madin-Darby Bovine Kidney ATCC CCL 22) isolated in our laboratory designated as MDBK-CLONE 4 was used as host cells for infection with *Eimeria tenella in vitro*. These cells were seeded into 35 mm petri dishes (containing 2—12 mm$^2$ glass coverslips) at a level of $2 \times 10^5$ cells per dish, irradiated 48 hours later with 1500 rads of gamma radiation, and infected (after an additional 24 hours) with freshly excysted *E. tenella* sporozoites at a parasite per host cell ratio of 3:1. Three hours after infecting all remaining extracellular sporozoites were removed by washing in fresh media. Forty-five hours post infection, the coverslips were rinsed in phosphate buffered saline pH 7.4, fixed with methanol, air dried and stained with Giemsa. By quantifying the percentage of cells with developing parasites it was determined that approximately 30—40% of the host cells contained at least one developing *E. tenella* parasite. In contrast, control cultures containing non-irradiated host cells had a maximum 8—15% host cells containing developing parasites, this represents at least a 2—3 fold increase in the number of cells with developing parasites when utilizing the irradiated host cell method. All cultures were incubated at 41°C, 5% $CO_2$.

Example 6 (Comparative)

Rat L-6 myoblast cells, $8\times10^6$ are seeded in a 150 cm² tissue culture flask using the same cell culture medium as in Example 1 and infected with $4\times10^7$ *T. cruzi* trypomastigotes (MERCK strain MERC2CD14). After 24 hours the cell culture medium is removed and replaced with the fresh cell culture medium. After 2 days the cell count is increased to $6.4\times10^7$. Many of the cells have overgrown the monolayer, sloughed off and dried. No extracellular parasites can be harvested.

Example 7 (Comparative)

In order to prevent overgrowing and sloughing off of cells, a 150 cm² flask is seeded with $3\times10^4$ rat L-6 myoblast cells. After 24 hours they are infected by addition of $3\times10^7$ *T. cruzi* trypomastigotes (Merck strain MERC2CD14). Culture medium is changed on days 2 and 4 after infection. Parasites are harvested on days 5—7. The total yield is $4\times10^8$ trypomastigotes.

**Claims**

1. A method for *in vitro* production of intracellular protozoa except Toxoplasma comprising:
   a) treating host cells to inhibit cell division, and
   b) after inhibition, infecting such host cells with protozoa.

2. A method according to claim 1 wherein step b) takes place only after at least time interval equivalent to the cell division time to synchronyze the cells.

3. A method according to claim 1 or 2 wherein the protozoa are Leishmania, Plasmodia, Theileria, Anaplasma, Trypanosoma, Coccidia or Babesia.

4. A method according to claim 1 wherein the cell division is inhibited by chemical treatment or by physical treatment.

5. A method according to claim 1 wherein the inhibition of cell division is effected by means of irradiation.

6. A method according to claim 5 wherein the irradiation level is that level effective to inhibit cell division but iffective to prevent the ability of the cells to serve as hosts when subsequently infected by protozoa.

7. A method according to claim 5 wherein the irradiation comprises gamma radiation.

**Patentansprüche**

1. Verfahren zur Herstellung von intrazellulären Protozoen ohne Toxoplasma, in vitro, durch:
   a) Behandeln von Wirtszellen, zur Inhibierung der Zellteilung, und
   b) nach der Inhibierung, Infektion dieser Wirtszellen mit Protozoen.

2. Verfahren nach Anspruch 1, bei dem die Stufe b) nur nach zumindest einem Zeitintervall erfolgt, das äquivalent zur Zellteilungs-Zeit ist, um die Zellen zu synchronisieren.

3. Verfahren nach Anspruch 1 oder 2, bei dem die Protozoen Leishmania, Plasmodia, Theileria, Anaplasma, Trypanosoma, Coccidia oder Babesia sind.

4. Verfahren nach Anspruch 1, bei dem die Zellteilung durch chemische Behandlung oder durch physikalische Behandlung inhibiert wird.

5. Verfahren nach Anspruch 1, bei dem die Inhibierung der Zellteilung durch Bestrahlung bewirkt wird.

6. Verfahren nach Anspruch 5, bei dem die Bestrahlungsdosis die Dosis ist, die zur Inhibierung der Zellteilung wirksam ist, jedoch unwirksam zur Verhinderung der Fähigkeit der Zellen ist, als Wirte zu dienen, wenn sie anschließend durch Protozoen infiziert werden.

7. Verfahren nach Anspruch 5, bei dem die Strahlung Gammastrahlung umfaßt.

**Revendications**

1. Procédé de production in vitro de protozoaires intracellulaires, sauf Toxoplasma, caractérisé en ce qu'il consiste:
   a) à traiter les cellules-hôtes, pour inhiber la division des cellules, et
   b) à infecter, après l'inhibition, cesdites cellules-hôtes avec les protozoaires.

2. Procédé selon la revendication 1, caractérisé en ce que le stade (b) a lieu, seulement après au moins un intervalle de temps équivalent au temps de division des cellules, afin de synchroniser les cellules.

3. Procédé selon la revendication 1 ou 2, caractérisé en ce que les protozoaires sont Leishmania, Plasmodia, Theileria, Anaplasma, Trypanosoma, Coccidia ou Babesia.

4. Procédé selon la revendication 1, caractérisé en ce que l'on inhibe la division cellulaire par un traitement chimique ou un traitement physique.

5. Procédé selon la revendication 1, caractérisé en ce que la division cellulaire est inhibée par irradiation.

6. Procédé selon la revendication 5, caractérisé en ce que le taux d'irradiation est le taux efficace pour inhiber la division des cellules mais inefficace pour empêcher les cellules de pouvoir servir de cellules-hôtes quand on les infecte ultérieurement avec des protozoaires.

7. Procédé selon la revendication 5, caractérisé en ce que l'irradiation comprend un rayonnement gamma.